# FASCICULE DE BREVET EUROPEEN

(11) **EP 0 680 336 B1**
(45) Date de publication et mention de la délivrance du brevet: **12.07.2000**
(21) Numéro de dépôt: 94905138.7
(22) Date de dépôt: 21.01.1994
(51) Int. Cl.: A61K 39/395

(54) **ANTICORPS MONOCLONAUX ANTI-LFA-1 POUR LA PREPARATION D'UN MEDICAMENT DESTINE A PREVENIR LE REJET DES GREFFES D'ORGANES**
MONOKLONALE ANTIKOERPER GEGEN LFA-1 ZUR HERSTELLUNG EINES ARZNEIMITTELS ZUR VORBEUGUNG DER TRANSPLANTATABSTOSSUNG VON ORGANEN
MONOCLONAL ANTI-LFA-1 ANTIBODIES FOR THE PREPARATION OF A MEDICAMENT INTENDED TO PREVENT THE REJECTION OF ORGAN TRANSPLANTS

(30) Priorité: 21.01.1993 FR 9300584
(43) Date de publication de la demande: 08.11.1995
(73) Titulaire: IMTIX-SANGSTAT, 69007 Lyon (FR)
(72) Inventeur: ALBERICI, Gilles, F-69009 Lyon (FR); CAUDRELIER, Pierre, F-69570 Dardilly (FR); HOURMANT, Maryvonne, F-44000 Nantes (FR); LE MAUFF, Brigitte, F-44300 Nantes (FR); SOULILLOU, Jean-Paul, F-44000 Nantes (FR)
(74) Mandataire: Bernasconi, Jean
(86) Numéro de dépôt international: FR9400071
(87) Numéro de publication internationale: WO9416728

(56) Documents cités:
- WO-A-89/04175
- US-A- 5 209 928
- TRANSPLANTATION vol. 52, no. 2 , Aoüt 1991 , BALTIMORE, MD, TATS-UNIS pages 291 - 296 B. LE MAUFF ET AL. 'Effect of anti-LFA1 (CD11a) monoclonal antibodies in acute rejection in human kidney transplantation.' cité dans la demande
- TRANSPLANTATION vol. 53, no. 4 , 1992 , BALTIMORE, MD, TATS-UNIS pages 840 - 849 P. BERLIN ET AL. 'Monoclonal antibodies against human T cell adhesion molecules. Modulation of immune function in nonhuman primates.' cité dans la demande
- M. ISOBE, Dans: 'Proc. of the International Congress of Immunology', Budapest, 23-28 aout 1992 (Ed. Hungarian Soc. for Immunol.), Springer, Berlin, 1992 cité dans la demande
- B. LEMAUFF et al., Dans: 'Proc. of the International Congress of Immunology', Budapest, 23-28 aout 1992 (Ed. Hungarian Soc. for Immunol.), Springer, Berlin,1992.
- TRANSPLANTATION vol. 55, no. 2 , Février 1993 , BALTIMORE, MD, TATS-UNIS pages 418 - 422 A. TALENTO ET AL. 'A single administration of LFA-1 antibody confers prolonged allograft survival.'
- COSIMI ET AL.: "In vivo effects of monoclonal antibody to ICAM-1 (CD54) in nonhuman primates with renal allografts", JOURNAL OF IMMUNOLOGY, , 15-06-1990, Vol. 144, no. 12, pages 4604 à 4612
- 8th Congress of the European Society for Organ Transplantation, September 2-6, 1997, Budapest, Hungary, abstract, page 8

## Description

Utilisation d'anticorps monoclonaux anti-LFA-1 pour la préparation d'un médicament destiné à prévenir le rejet des greffes d'organes solides.

La présente invention concerne l'utilisation d'anticorps monoclonaux dirigés contre la molécule LFA-1 humaine pour la préparation d'un médicament de traitement de base de la prévention du rejet des greffes d'organes solides, à savoir le rein, le coeur, le poumon, le foie et des glandes endocrines chez l'homme, destiné à une administration sous forme d'une dose initiale avant le rétablissement de la continuité vasculaire du greffon implanté, suivie d'une dose quotidienne pendant une période débutant sensiblement le jour de la greffe et inférieure ou égale à dix jours.

La dernière décennie a vu une amélioration des résultats des transplantations d'organes, notamment de reins, se traduisant par une amélioration de la survie des patients et des greffons. Avant 1981, l'utilisation d'Azathioprlne (AZA) et de Prednisone (P) constituait l'immunosuppression prophylactique de base en matière de transplantation rénale, malgré un taux relativement faible de survie du greffon. L'apparition de la Cyclosporine A (CsA) a permis d'améliorer significativement la survie des greffons, approximativement de 90 % à un an (in "Annual Report of United Network for Organ Sharing" 1989, UNOS Eds. Richmond - J.M. Cecka et al., The UNOS scientific renal transplant registry, In "Clinical transplants" 1989 : 1-8 UCLA Eds. Los Angeles) avec une survie prolongée des patients (environ 95 % sur un an), mais avec comme contrepartie des problèmes de toxicité non négligeables. On a également inclus des anticorps antilymphocytaires polyclonaux et monoclonaux dans des protocoles de prophylaxie et de traitement curatif de rejet.

On a pu montrer de faibles taux de rejet précoce des greffons par un traitement en deux temps comprenant initialement l'utilisation d'anticorps polyclonaux antilymphocytaires puis de CsA à titre d'immunosuppresseur principal de maintenance (A.J. Richardson et al., Transplant Internat 1990 ; 3 : 26-31 - B.G. Sommer et al., Transplantation 1987 ; 43 : 85-90).

L'arrivée des anticorps monoclonaux (mAb) à spécificité déterminée a permis d'envisager une action plus précise en termes d'induction d'immunosuppression et de traitement des épisodes de rejet. Jusqu'à présent, seul l'anticorps OKT3 (mAb anti-CD3) a reçu une autorisation de mise sur le marché pour le traitement curatif des épisodes de rejet de greffe rénale (Orthoclone OKT®3, Product Information, Physicians' Desk Reference, 43ème Edition (Medical Economics Company Inc., N.J. Oradell, 1989, pages 1500-1501). On ne dispose cependant que de peu de preuves de son efficacité pour la prévention des rejets de greffe de rein (en combinaison avec une immunosuppression chimique). Pour l'instant, aucun anticorps monoclonal n'a été reconnu pour cette indication.

La molécule LFA-1 (molécule de fonction lymphocytaire) est une intégrine qui appartient au complexe d'adhésion lymphocytaire qui intervient dans les phénomènes d'adhésion cellulaire et de communication intercellulaire et qui accroît notamment les interactions entre lymphocytes auxiliaires et leurs cellules cibles. Cette famille de produits inclut les molécules Mac1, LFA-1 et Gp150,95 qui présentent une chaîne bêta commune de 95 kD et se distinguent les unes les autres par leur chaîne alpha. La protéine LFA-1 ou CD11a/CD18 est un dimère de 180 kD présent à la surface des cellules de moelle osseuse (lignées leucocytaires), des lymphocytes T, des cellules NK, des polynucléaires et des macrophages/monocytes. In vitro, des anticorps monoclonaux dirigés contre LFA-1 inhibent la plupart des activités des cellules T.

L'anticorps anti-LFA-1 a été utilisé chez l'enfant pour la greffe de moelle osseuse HLA incompatible (A. Fischer et al., Lancet 1986 ; ii, (8515) : 1058-1061 - N. Perez et al., Bone Marrow Transplant 1989 ; 4 : 379-384). Les anticorps anti-LFA-1 ont également été utilisés chez des patients leucémiques adultes pour prévenir un rejet de greffe de moelle osseuse à HLA et déplétée en cellules T (D. Maraninchi et al., Bone Marrow Transplant 1989 ; 4 : 147-150). Ils ont également été utilisés pour traiter 10 patients présentant une réaction aiguë du greffon contre l'hôte résistant aux traitements par les stéroïdes (A.M. Stoppa et al., Transplant Int. 1991 ; 4 : 3-7).

En matière de transplantation rénale, les anticorps monoclonaux anti-LFA-1 ont été utilisés chez sept patients pour traiter des épisodes de rejet de greffe aigu (B. Le Mauff et al., Transplantation 1991 ; 52, (2) : 291-296). Il s'agit de l'anticorps monoclonal dénommé 25.3. La tolérance était bonne chez les six patients qui ont reçu plus d'une administration de cet anticorps. Des infections ont été rapportées chez deux patients. Cependant, un seul patient, probablement celui faisant l'épisode de rejet le plus faible, a recouvré sa fonction rénale avant rejet et un traitement de secours a dû être mis en oeuvre chez cinq d'entre eux. En conclusion cet anticorps a été considéré comme inefficace pour traiter le rejet aigu survenant au décours des greffes de rein. L'application à la prévention du rejet n'a pas été étudiée.

P.J. Berlin et al., Transplantation, Vol 53, n° 4, 1992, Baltimore MD, USA, ont décrit une certaine efficacité de l'administration d'anticorps anti LFA-1 pour bloquer l'activité des cellules T pendant le rejet d'une allogreffe cutanée chez le singe et une légère prolongation de la survie du greffon avant le rejet. Le seul greffon survivant à trois mois correspond à un traitement associant un anti CD11 à un anti-CD2.

Des anticorps monoclonaux dirigés contre ICAM-1, le ligand naturel de LFA-1, ont montré certains résultats dans le rejet de greffe de rein chez des primates (B.A. Cosimi et al. Leukocyte Adhesion Molecules 1989 : 274). Les auteurs suggèrent qu'un traitement combinant les anticorps anti ICAM-1 et anti LFA-1 pourrait être plus efficace (B.A. Cosimi et al. J. of Immunol., 1990, vol 144, No. 12, 4604-4612).

M. Isobe, Proceedings of Int. Congress of Immunol. Budapest, Août 23-28, 1992 (Ed Hungarian Soc. for Immunol.) Springer, Berlin, 1992, 554, W-90-19, suggère également un effet de synergie des monoclonaux anti ICAM-1 et anti LFA-1 dans la tolérance d'allogreffes chez le rongeur.

Le Mauff et al., dans Proc. of the International Congres of Immunology, Budapest, 23-28 août 1992 (Ed. Hungarian Soc. for Immunol., Springer, Berlin, 1992, 603, Abstract W-97-30 observent un allongement de la survie de greffes hétérotopiques de coeur chez la souris par un anticorps monoclonal anti-LFA-1. Ces modèles de greffes hétérotopiques ne permettent cependant pas d'étudier le caractère fonctionnel des greffons et la transposition de ce modèle chez l'homme n'est pas non plus possible, notamment en raison de la spécificité anti-souris.

WO 89/04175 décrit un anticorps monoclonal capable de reconnaître spécifiquement un épitope particulier de l'antigène LFA-1, ce qui lui donne des propriétés particulières. Il évoque une éventuelle utilité dans le traitement du rejet des greffes, sans envisager le problème de la prévention.

L'état de la technique, tout en reconnaissant aux anticorps anti-LFA-1, certaines propriétés utiles, ne suggère donc pas d'utiliser ces anticorps pour la prévention du rejet de greffes d'organes solides, sauf sous la forme d'associations avec d'autres anticorps impliqués également dans les phénomènes d'adhésion cellulaire.

La demanderesse a maintenant trouvé qu'il était possible de prévenir le rejet des greffes d'organes solides, tels que le rein, par l'administration d'anticorps monoclonal dirigé contre la molécule LFA-1 (CD11a/CD18) humaine et ce, sans association avec d'autres anticorps ni avec la cyclosporine A.

Elle a également trouvé que l'efficacité de cette utilisation était grandement subordonnée à un protocole original d'administration.

La présente invention a donc pour objet l'utilisation d'anticorps monoclonaux dirigés contre la molécule LFA-1 humaine pour la préparation d'un médicament de traitement de base de la prévention du rejet des greffes d'organes solides, à savoir le rein, le coeur, le poumon, le foie et des glandes endocrines chez l'homme, destiné à une administration sous forme d'une dose initiale avant le rétablissement de la continuité vasculaire du greffon implanté, suivie d'une dose quotidienne pendant une période débutant sensiblement le jour de la greffe et inférieure ou égale à dix jours.

Par anticorps dans le sens de la présente invention on entend les anticorps humains, non humains, par exemple murins, humanisés, chimériques, recombinants, ou autres, ainsi que les dérivés d'anticorps, fragments, etc. Tous ces anticorps, y compris les dérivés, peuvent être préparés par les méthodes classiques.

On peut caractériser notamment les anticorps monoclonaux utiles dans l'invention par le fait qu'ils réagissent avec :
- les lymphocytes T et B, les monocytes, les macrophages et les polymorphonucléaires ;
- 60 % environ des thymocytes et prothymocytes ;
- des lignées de cellules T (par exemple lignées MOLT-4, HPB-ALL et CEM) ;
- la lignée KG1, isolée à partir d'une leucémie myéloïde aiguë.

Les anticorps monoclonaux selon l'invention ont pour objectif de bloquer la molécule LFA-1 et de réduire ainsi les interactions intercellulaires. L'anticorps monoclonal doit inhiber l'adhésion et les fonctions effectrices des cellules T et des cellules NK.

Outre les propriétés de liaison définies ci-dessus avec les différentes classes cellulaires, les anticorps monoclonaux selon l'invention ont avantageusement tout ou partie des propriétés suivantes in vitro :
- inhibition de la réaction lymphocytaire mixte et partiellement des proliférations induites par la phytohémagglutinine (PHA) ;
- inhibition de la cytotoxicité T-dépendante et de la cytotoxicité NK ;
- altération du pouvoir d'adhésion des polynucléaires sur le verre ;
- absence de réaction avec la membrane des leucocytes d'enfants souffrant d'une immunodéficience congénitale en récepteur LFA-1 CR3 et complexe Gp 150, 95 ;
- inhibition de la fixation de la composante C3bi du complément sur son récepteur CR3 ;
- inhibition de l'activité productrice d'anticorps des cellules T helper spécifiques de l'antigène ;
- inhibition partielle de la prolifération des cellules T induite par l'antigène ;
- absence de prolifération cellulaire et de production de TNF-α ;
- diminution (à forte concentration) des réponses prolifératives induites par les mitogènes PMA, ConA, PWM et OKT3 en solution ;
- diminution des cellules blastiques et de l'expression de la chaîne a du récepteur de l'IL-2 (CD25) ;
- diminution des réponses prolifératives des suspensions cellulaires appauvries en monocytes stimulées par SEB ou des cellules B alloréactives traitées à la mitomycine (MLR).

De préférence, l'anticorps monoclonal sera une IgG1 de souris et notamment anticorps 25.3 (référence interne 25.3.1.19.3B7), également désigné 25.3.1 dans le Leucocyte Typing III, Oxford 21-26 Sept 1986, déposé auprès de la collection ECACC sous la référence 92 120 309 le 3 décembre 1992.

L'anticorps monoclonal destiné à prévenir le rejet de greffes d'organes solides chez un receveur est de préférence administré à raison d'environ 1 à 50 mg/jour, notamment d'environ 15 à 20 mg/jour ; de préférence sur une période allant de 1 à 30 j., notamment de 3 à 14 ; de préférence par perfusion, notamment de l'ordre de 30 min.

L'invention a également pour objet un médicament comprenant à titre de principe actif au moins un anticorps monoclonal dirigé contre la molécule LFA-1 humaine en solution isotonique à 1 mg/ml environ dans un véhicule approprié.

L'invention a également pour objet un médicament comprenant à titre de principe actif au moins un anticorps monoclonal dirigé contre la molécule LFA-1 humaine en solution aqueuse isotonique pour perfusion et des agents de stabilisation tels que le tris (hydroxyméthyl) aminométhane et le Tween 80®.

De préférence, pour une solution d'un litre, le médicament en solution comprend de 0,1 à 10 g, notamment 1 g, environ, d'anticorps monoclonal, et de 0,1 à 10 g, notamment 2,4 g, environ, de tris (hydroxyméthyl) aminométhane ou analogue, et de 0,1 à 10 pour mille de Tween® 80, notamment 2 pour dix mille, dans un soluté isotonique.

Les médicaments selon l'invention comprennent de préférence un anticorps monoclonal dirigé contre la chaîne alpha de la molécule LFA-1 et plus particulièrement un anticorps monoclonal tel que défini ci-dessus.

L'invention a encore pour objet un kit destiné à l'administration simultanée, séparée ou étalée dans le temps, en vue de la prévention des rejets de greffes d'organes solides, comprenant :
- un médicament dont le principe actif est au moins un anticorps dirigé contre la molécule LFA-1, de préférence un anticorps monoclonal du type décrit ci-dessus,
- et au moins un autre composé actif en matière de prévention du rejet des greffes d'organes, notamment choisi parmi les composés suivants :
   . le Tacrolimus (Fk 506),
   . l'Azathioprine,
   . un stéroïde, tel que la Prednisone ou un corticostéroïde équivalent,
      la cyclosporine A.

L'ivention a encore pour objet un procédé pour la prévention du rejet des greffes d'organes solides tels que le rein, dans lequel on administre au moins un anticorps monoclonal anti-LFA-1 tel que décrit ci-dessus et éventuellement au moins un autre agent choisi parmi ceux du kit ci-dessus. De préférence, l'anticorps monoclonal est administré à raison de 1 à 50 mg/jour, notamment d'environ 20 mg/jour ; de préférence par perfusion, notamment de l'ordre de 30 min.

La présente demande décrit également un procédé pour la prévention du rejet des greffes d'organes solides , tels que le rein, caractérisé en ce que l'on administre une dose initiale d'un anticorps anti-LFA-1 peu de temps, notamment dans les deux heures, par exemple une heure, avant le rétablissement de la continuité vasculaire du greffon implanté, puis des doses quotidiennes pendant environ 9 jours.

La dose initiale est de préférence de 30 mg (2 x 15 mg) et les doses suivantes, chez l'adulte, sont avantageusement de l'ordre de 15 mg, administrées de préférence par perfusion dans une veine périphérique.

Le dosage, rapporté au poids, est plus important chez l'enfant.

Le procédé de prévention est remarquable en ce qu'il peut-être avantageusement conduit sans autre médication majeure, en particulier sans autre anticorpsmonoclonal ou gamma globulines anti-T, et sans cyclosporine.

De façon avantageuse un traitement à la cyclosporine peut être mis en oeuvre le 9ème ou 10ème jour au moment de l'arrêt du traitement anti-LFA-1.

Le procédé peut, par ailleurs, être associé au traitement par corticoïdes et azathioprine habituel.

L'invention va être maintenant décrite plus en détail à l'aide d'un mode de réalisation de l'invention.

L'anticorps monoclonal 25.3 est récolté après culture en fermenteurs dans un milieu sans protéine. Les surnageants hebdomadaires sont concentré puis purifiés par trois étapes chromatographiques sur Q-Sepharose® FF, puis S-Sepharose FF®, puis Q-Sepharose® FF.

On prépare des flacons de 5 ml d'anticorps 25.3 en concentration de 1 mg/ml :

| | |
|---|---|
| - anticorps | 5 mg, |
| - tris (hydroxyméthyl) aminométhane | 12,1 mg, |
| - NaCl | 43,5 mg, |
| - Tween 80® | 1 mg, |
| - Eau pour injection qs | 5 ml, |

Des traitements immunosuppresseurs peuvent être associés :
- Cyclosporine A (SANDIMMUM ®) : à raison de 8 à 10 mg/kg/jour par voie orale à partir du 9ème jour après la greffe ; en une à deux administrations par jour pendant une durée indéterminée ; ou par voie intraveineuse à raison de 2 à 4 mg/kg/jour.
- Azathioprine (Imurel®) : à raison de 2 mg/kg/j à partir du jour de la greffe, par voie orale ou intraveineuse, pour une dose totale de 25 mg ; administration en une fois ; la dose journalière peut aller de 2 à 2,5 mg/kg.
- Stéroïdes : à raison de 1 mg/kg/j de Prednisone ou d'un corticostéroïde équivalent, aux jours 1 à 5, par voie orale ; à partir du 6ème jour, on diminue la dose tous les 5 jours de 10 mg, puis arrêt total au 45ème jour.

Des traitements divers peuvent être associés si besoin est ; par exemple :
- antifongique oral tel qu'amphotéricine B (Fungizone®) à raison de 1.5 mg/jour pendant un mois,
- antibiotique tel que Sulfaméthoxazole-Triméthoprime (Bactrim®) à raison de 800 mg/jour pendant 3 mois,
- traitement contre la fièvre : par exemple paracétamol par voie orale à raison de 650 mg, selon les besoins,
- traitement contre le prurit à l'aide d'un antihistaminique tel que la diphénhydramine à raison de 50 mg par voie orale, selon les besoins.

### Expérience clinique en prophylaxie du rejet de greffe de rein.

Un essai clinique de phase II, monocentrique, ouvert, non randomisé, est réalisé chez quinze patients devant subir une 1ère transplantation rénale.

Le premier patient a été inclus le 25 mai 1992.Tous les patients ont été suivis trois mois post-greffe.

Les cinq premiers patients (patients 1 à 5) ont reçu 20 mg/jour d'anticorps anti-LFA1 25.3 pendant 10 jours. Au vu des résultats cliniques et biologiques obtenus dans ce premier groupe, les cinq patients suivants (patients 6 à 10) ont reçu 15 mg/jour. Les cinq derniers patients ont reçu 10 mg/jour (patients 11 à 15).

### Critères d'inclusion.

Première greffe de rein de cadavre, receveur entre 18 et 60 ans, donneur entre 10 et 60 ans, durée d'ischémie froide < 48 h, et obtention d'un consentement de participation signé.

### Protocole de traitement utilisé.

L'anticorps est perfusé par voie intraveineuse sur une durée de 30 minutes, des jours J1 (jour de la transplantation) à J10.

Par rapport au protocole standard de triple thérapie utilisé habituellement dans le centre, l'introduction de la cyclosporine est retardée au 9ème jour post-transplantation afin d'atteindre les taux résiduels recommandés lors de l'interruption de l'anti-LFA1 (à J10). L'azathioprine est administrée à la dose de 2 mg/kg/jour à partir de J1 à 5, puis réduit de 10 mg tous les 5 jours à partir de J6 et interrompu à J45 post-transplantation.

### Tolérance aux injections.

La tolérance immédiate aux injections est excellente : tous les patients ont reçu la totalité du traitement. Il n'a pas été observé de différence entre les groupes (20 ou 15 mg) en terme de tolérance à l'injection d'anti-LFA-1.

Tolérance biologique : la moitié des patients s'est immunisé contre l'anticorps mais l'immunisation est toujours survenue après le dernier jour de traitement (entre J12 et J30).

| Evolutian clinique - Tableau résumé | | | | |
|---|---|---|---|---|
| Nombre de patients | | 5 | 5 | 5 |
| Nombre de patients évaluables | | 5 | 5 | 4 * |
| Anti-LFA1 (mg/jour) 10 jours | | 20 | 15 | 10 |
| Suivi moyen (jours) | | 90 | 90 | 90 |
| Arrêt de traitement | | 0 | 0 | 0 |
| Immunisation | à J15 | 3/5 | 0/5 | 2/4 |
| | à J30 | 3/5 | 2/5 | 1/4 |
| Episodes de rejet | avant J10 | 0 | 0 | 0 |
| | de J10 à J30 | 0 | 0 | 0 |
| | de J30 à J90 | 1 | 3 | 2 |
| | (nb de patients) | (1) | (2) | (2) |
| Episodes infectieux | | 0 | 3 (2 CMV; 1 Candidose | 4 (2 CMV 1 Candidose 1 Pvelonephrite) |
| Créatinémie (micromol/l) Moyenne ± s.e.m. (n=5) | J1 | 545 ± 221 | 516 ± 137 | 617 ± 135 |
| | J30 | 148 ± 20 | 229 ± 109 | 140 ± 38 |
| | J90 | 167 ± 48 | 186 ± 118 | 133 ± 22 |

| | | | | |
|---|---|---|---|---|
| * 1 cas de thrombose artérielle immédiate | | | | |

Tous les patients sont en vie et ont une greffe fonctionnelle à 3 mois post-greffe à l'exception d'un patient du groupe 3 qui a perdu son greffon par thrombose artérielle immédiate sans rapport avec un phénomène de rejet.

Aucun épisode de rejet sous traitement anti-LFA1 n'a été observé. Six patients ont présenté au moins un épisode de rejet (entre le premier et le deuxième mois). Ces épisodes de rejet ont tous été résolus par des traitements conventionnels.

Le suivi biologique de l'étude a comporté l'étude de plusieurs paramètres.

La mesure des taux d'anticorps circulants a été effectuée par un test immunoenzymatique détectant les immunoglobulines (Ig) de souris dans le sérum en référence à une courbe étalon de 25.3 purifié. Les deux groupes 20 et 15 mg présentent des taux élevés de 25.3 circulants atteignant en moyenne 12 et 10 µg/ml à la fin du traitement. La différence entre les premier et deuxième groupes n'est pas statistiquement significative, et le 25.3 est encore détectable dans les deux groupes à J14, voire J20 pour certains patients. Dans le troisième groupe les taux étaient nettement plus faibles et dispersés mais néanmoins significatifs quatre jours après la dernière injection.

L'anticorps n' entraine pas de modification majeure du nombre de cellules circulantes, il n'est donc pas déplétant. Par contre les cellules circulantes et en particulier les lymphocytes et les monocytes sont recouverts de 25.3 de façon saturante. En effet, lorsque les cellules des patients sont étudiées en cytofluorométrie, le marquage obtenu avec un anticorps anti-souris couplé à la fluorescéines est de même intensité avec ou sans marquage préalable in vitro avec une quantité saturante de 25.3 (20 µg/mg). Cette saturation s'observe encore à J14 pour 3/5 des patients du groupe I et pour tous les patients du groupe II.

La présence de l'anticorps circulant et la saturation des cellules s'accompagne d'un blocage fonctionnel des cellules mis en évidence au niveau des capacités d'adhésion des lymphocytes T. Il est connu que les molécules LFA1/ICAM-1 sont impliquées dans l'adhésion hétérotypique des lymphocytes T à des cellules cibles lymphoblastoïdes B.

Il a donc été mis au point un test d'adhésion entre les lymphocytes T du sang circulant et une lignée B (Daudi). Les lymphocytes sont isolés sur gradient de Ficoll®, puis marqués avec un anti-CD3 couplé à la phycoérythrine (7 µg/ml) et la lignée B est marquée en immunofluorescence indirecte avec un anticorps monoclonal anti-Daudi, puis un anticorps anti-souris couplé à la fluorescéine. Les cellules reprises en milieu RPMI sont mises en présence dans un rapport 1:1 (cellules mononuclées : Daudi) et incubées soit à 4°C, soit à 37°C (avec et sans addition de 25.3, 15 µg/ml). La réaction est arrêtée par addition de 2 volumes de milieu froid et les cellules sont conservée à 4°C jusqu'à l'analyse en cystométrie dans les 2 h qui suivent. Lors de l'analyse, on mesure parmi les cellules CD3+ celles qui sont doublement positives. Ces événements doublement positifs correspondent à des conjugués T-Daudi comme le confirme l'augmentation apparente de taille (x2 ou x3) des cellules CD3+ "doublement" marquées. Le nombre de conjugués correspond au pourcentage de conjugués obtenus à 37°C, moins le pourcentage obtenu en présence de 25.3 à 15 µg/ml.

Les résultats obtenus avec les cellules des patients sont exprimés sous la forme d'un index d'adhésion, c'est-à-dire rapportées aux valeurs obtenues dans la même expérience avec des cellules de sujets témoins (donneurs sains de façon à normaliser les variations inter-essais).

Dans tous les groupes de patients, l'index d'adhésion est complètement effondré pendant le traitement, puis remonte progressivement entre J20 et J30. Un seul patient, du groupe I, a récupéré une adhésion normale à J15 et présente par ailleurs une immunisation importante contre le 25.3, avec présence d'anticorps anti-idiotypes. La récupération des capacités d'adhésion est en fait contemporaine de la désaturation des cellules circulantes.

On a aussi, au cours du traitement, analysé l'expression de la molécule LFA1 sur les lymphocytes des patients par cytofluorométrie. On a pu observer une diminution de l'intensité de fluorescence du CD11a révélé par le 25.3, pouvant atteindre 50 %, et ce dans les deux groupes. Cette modulation commence dès la première injection mais est plus importante entre J10 et J15. Elle s'accompagne d'une diminution tout à fait parallèle de l'expression du CD18, c'est-à-dire de la chaîne β de la molécule LFA1.

Sept des quatorze patients suivis ont présenté une immunisation, sans effets durant le traitement. Seuls quatre patients ont montré, après le traitement, des anticorps Ig6 de souris détectables au delà d'une dilution au 1/300.

En résumé le 25.3 est un anticorps non déplétant qui, aux doses utilisées, sature les sites LFA-1 présents sur les cellules circulantes et ainsi entraîne un blocage des capacités d'adhésion lymphocytaire LFA-1 dépendantes. Le traitement peut s'accompagner d'une modulation de l'expression cellulaire de la molécule LFA1. Il est capable d'induire une réponse anticorps souvent faible mais parfois (1 cas/10 dans cette étude) importante, après la période de traitement, avec présence d'anti-idiotypes.

## Revendications

1. Utilisation d'anticorps monoclonaux dirigés contre la molécule LFA-1 humaine pour la préparation d'un médicament de traitement de base de la prévention du rejet des greffes d'organes solides, à savoir le rein, le coeur, le poumon, le foie et des glandes endocrines chez l'homme, destiné à une administration sous forme d'une dose initiale avant le rétablissement de la continuité vasculaire du greffon implanté, suivie d'une dose quotidienne pendant une période débutant sensiblement le jour de la greffe et inférieure ou égale à dix jours.

2. Utilisation selon la revendication 1, caractérisée en ce que l'anticorps monoclonal est dirigé contre la chaîne alpha de la molécule LFA-1.

3. Utilisation selon la revendication 1 ou 2, caractérisée en ce que l'anticorps monoclonal réagit avec :
- les lymphocytes T et B, les monocytes, les macrophages et les polymorphonucléaires ;
- 60 % environ des thymocytes et prothymocytes ;
- des lignées de cellules T ;
- la lignée KG1.

4. Utilisation selon l'une quelconque des revendications 1 à 3, caractérisée en ce que l'anticorps monoclonal est une IgG1 de souris.

5. Utilisation selon la revendication 4, caractérisée en ce que l'anticorps monoclonal est l'anticorps 25.3 déposé auprès de la collection ECACC sous la référence 92 120 309.

6. Utilisation selon l'une quelconque des revendications 1 à 5 pour la préparation d'un médicament destiné à prévenir le rejet des greffes d'organes solides chez un receveur auquel le médicament est administré à raison d'environ 1 à 50 mg d'anticorps/jour, notamment d'environ 20 mg/jour.

7. Utilisation selon la revendication 6 pour la préparation d'un médicament destiné à prévenir le rejet des greffes d'organes chez un receveur auquel le médicament est administré pendant une période comprise entre 3 et 10 jours.

8. Utilisation selon la revendication 6 ou 7, pour la préparation d'un médicament destiné à prévenir le rejet des greffes d'organes solides chez un receveur auquel le médicament est administré par perfusion, notamment de l'ordre de 30 minutes.

9. Utilisation selon l'une des revendications 6 à 8, pour la préparation d'un médicament destiné à prévenir le rejet d'organes chez un receveur auquel le médicament est administré avec une dose de 30 mg avant la revascularisation du greffon puis une dose journalière de 15 mg.

## Claims

1. Use of monoclonal antibodies directed against the human LFA-1 molecule for preparing a basic medical treatment for preventing the rejection of transplants of solid organs, namely the kidney, heart, lung, liver and endocrine glands in humans, intended to be administered in the form of an initial dose before the vascular continuity of the implanted graft is restored, followed by a daily dose for a period starting substantially on the day of the transplant and less than or equal to ten days.

2. Use according to claim 1, characterised in that the monoclonal antibody is directed against the alpha chain of the LFA-1 molecule.

3. Use according to claim 1 or 2, characterised in that the monoclonal antibody reacts with:
- the T and B lymphocytes, the monocytes, the macrophages and polymorphonuclear leucocytes;
- about 60% of the thymocytes and prothymocytes;
- the T cell lines;
- the KG1 line.

4. Use according to any of claims 1 to 3, characterised in that the monoclonal antibody is a mouse IgG1.

5. Use according to claim 4, characterised in that the monoclonal antibody is antibody 25.3 deposited at the ECACC collection under reference number 92 120 309.

6. Use according to any one of claims 1 to 5 for preparing a medicament intended to prevent the rejection of solid organ transplants in a recipient to whom the medicament is administered in an amount of about 1 to 50 mg of antibody per day, in particular about 20 mg/day.

7. Use according to claim 6 for preparing a medicament intended to prevent the rejection of organ transplants in a recipient to whom the medicament is administered for a period of between 3 and 10 days.

8. Use according to claim 6 or 7, for preparing a medicament intended to prevent the rejection of solid organ transplants in a recipient to whom the medicament is administered by perfusion, in particular for a period of the order of 30 minutes.

9. Use according to one of claims 6 to 8, for preparing a medicament intended to prevent the rejection of organs in a recipient to whom the medicament is administered in a dose of 30 mg before the revascularisation of the transplant, followed by a daily dose of 15 mg.

## Patentansprüche

1. Verwendung monoklonaler Antikörper, die gegen das menschliche LFA-1-Molekül gerichtet sind, zur Herstellung eines Medikamentes für die Unterstützungsbehandlung zur Vorbeugung der Abstoßung von Transplantationen solider Organe, insbesondere Niere, Herz, Lunge, Leber und endokrine Drüsen, beim Menschen, das zur Verabreichung in Form einer Anfangsdosis vor der Wiederherstellung der Blutgefäßkontinuität des implantierten Transplantats, gefolgt von einer täglichen Dosis während eines Zeitraums, der genau am Tag der Transplantation beginnt und weniger als oder zehn Tage dauert, ausgelegt ist.

2. Verwendung nach Anspruch 1, dadurch gekennzeichnet, dass der monoklonale Antikörper gegen die Alpha-Kette des LFA-1-Moleküls gerichtet ist.

3. Verwendung nach Anspruch 1 oder 2, dadurch gekennzeichnet, dass der monoklonale Antikörper mit:
• T- und B-Lymphozyten, Monozyten, Makrophagen und polymorphonukleären Zellen;
• etwa 60% der Thymozycen und Prothymozyten:
• T-Zelllinien;
• der KG1-Linie
reagiert.

4. Verwendung nach einem der Ansprüche 1 bis 3, dadurch gekennzeichnet, dass der monoklonale Antikörper ein Maus-IgG1 ist.

5. Verwendung nach Anspruch 4, dadurch gekennzeichnet, dass der monoklonale Antikörper der Antikörper 25.3 ist, hinterlegt bei der ECACC-Sammlung unter der Bezugsnummer 92 120 309.

6. Verwendung nach einem der Ansprüche 1 bis 5 zur Herstellung eines Medikamentes, das zur Vorbeugung der Abstoßung von Transplantationen solider Organe bei einem Empfäriger ausgelegt ist, an den das Medikament zu etwa 1 bis 50 mg Antikörper/Tag, insbesondere etwa 20 mg/Tag verabreicht wird.

7. Verwendung nach Anspruch 6 zur Herstellung eines Medikamentes, das zur Vorbeugung der Abstoßung von Transplantationen solider Organe bei einem Empfänger ausgelegt ist, an den das Medikament während eines Zeitraums von 3 bis 10 Tagen verabreicht wird.

8. Verwendung nach Anspruch 6 oder 7 zur Herstellung eines Medikamentes, das zur Vorbeugung der Abstoßung von Transplantationen solider Organe bei einem Empfänger ausgelegt ist, an den das Medikament durch Perfusion, insbesondere während etwa 30 Minuten, verabreicht wird.

9. Verwendung nach einem der Ansprüche 6 bis 8 zur Herstellung eines Medikamentes, das zur Vorbeugung der Abstoßung von Organen bei einem Empfänger ausgelegt ist, an den das Medikament in einer Dosis von 30 mg vor der Revaskularisierung des Transplantats und dann in einer täglichen Dosis von 15 mg verabreicht wird.
